# EUROPEAN PATENT APPLICATION

(11) **EP 2 218 721 A1**
(43) Date of publication of application: **18.08.2010**
(21) Application number: 09152526.1
(22) Date of filing: 11.02.2009
(51) Int. Cl.: C07D 487/04, A61K 31/4985, A61P 3/08

(54) **Novel salts of sitagliptin**

(71) Applicant: LEK Pharmaceuticals d.d., 1526 Ljubljana (SI)
(72) Inventor: The designation of the inventor has not yet been filed

(57) **Abstract**

The present invention relates to novel pharmaceutically acceptable salts of sitagliptin, to processes for their preparation and to pharmaceutical compositions containing them.

## Description

### Field of the Invention

The present invention relates to novel pharmaceutically acceptable salts of sitagliptin, to processes for their preparation and to pharmaceutical compositions containing them.

### Background of the Invention

The compound (2R)-4-oxo-4-[3-(trifluoromethyl)-5,6.dihydro[1,2,4]-triazolo[4,3-a]pyrazin-7(8*H*)-yl]-1-(2,4,5-trifluorophenyl)butan-2-amine, also named sitagliptin (Formula I) has been shown to act as an inhibitor of dipeptidyl peptidase-IV (DPP-IV).

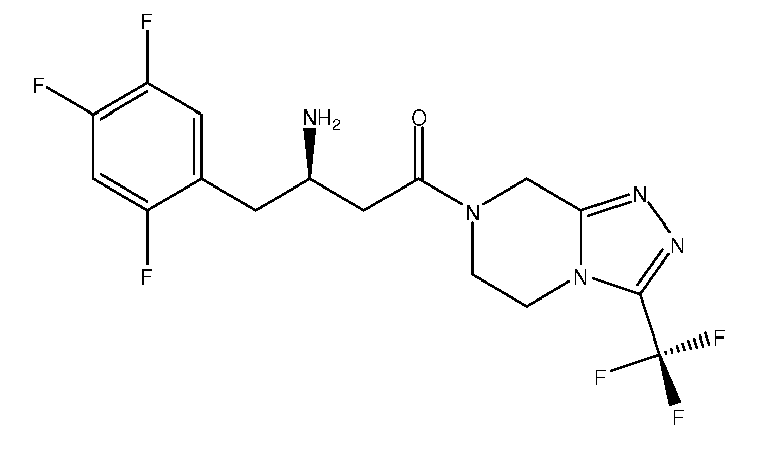

WO 2003/004498 generally relates to inhibitors of the dipeptidyl peptidase-IV which are useful in the treatment or prevention of diseases in which the dipeptidyl peptidase-IV enzyme is involved, such as diabetes and particularly type 2 diabetes, and specifically discloses sitagliptin. Examples of salts for the general class of compounds are generally referred to, such as salts prepared from pharmaceutically acceptable non-toxic acids. WO 2003/004498 is silent as to the preparation of and the nature of specific crystal forms of salts.

WO 2005/003135 describes dihydrogenphosphate salt of the dipeptidyl peptidase-IV inhibitor sitagliptin and crystalline hydrates thereof, in particular a crystalline monohydrate. In WO 2005/003135 it is said that sitagliptin dihydrogenphosphate salt and crystalline hydrates have advantages in the preparation of pharmaceutical compositions, such as ease of processing, handling, and dosing. In particular, they exhibit improved physical and chemical stability, such as stability to stress, high temperatures and humidity, as well as improved physicochemical properties, such as solubility and rate of solution.
W02005/020920 describes the crystalline anhydrate Form I, Form II and Form III as well as solvates of the sitagliptin dihydrogenphosphate salt.
W02005/030127 describes novel crystalline anhydrate Form IV of sitagliptin dihydrogenphosphate salt.
W02006/033848 describes the amorphous sitagliptin dihydrogenphosphate salt.
W02005/072530 describes crystalline hydrochloric acid, benzenesulfonic acid, p-toluenesulfonic acid, 10-camphorsulfonic acid, and tartaric acid salts of sitagliptin and hydrates thereof.
W02007/035198 describes sitagliptin dodecylsulfate salt, in particular, a crystalline anhydrate form thereof.
Salts often improve physical and biological characteristics of mother compounds without modifying primary pharmacological activity, based on mechanism of action.
Thus there is a continuing need to obtain new salts of sitagliptin having improved physical and/or chemical properties. The present invention satisfies this need by providing new salts of sitagliptin with enhanced solubility in water or aqueous media as an essential property of active pharmaceutical ingredients determining the performance of pharmaceutical formulation.

### Summary of the invention

The present invention provides the following items including main aspects and preferred embodiments, which respectively alone and in combination particularly contribute to solving the above object and eventually provide additional advantages:
(1) Sitagliptin salt with a pharmaceutically acceptable acid, the pharmaceutically acceptable acid being selected from the group consisting of D-glucuronic acid, L-glucuronic acid, glutaric acid, sulfuric acid, L-lactic acid, D-lactic acid, ethanesulfonic acid, oxalic acid, acetic acid, L-mandelic acid, D-mandelic acid, capric acid, benzoic acid, hippuric acid, trans-cinnamic acid, malonic acid, citric acid, 1-hydroxy-2-naphtolic acid, crotonic acid and ascorbic acid, and hydrates and solvates of said salt.
(2) Sitagliptin salt according to item (1), wherein the pharmaceutically acceptable acid is selected from the group consisting of D-glucuronic acid, L-glucuronic acid, glutaric acid, sulfuric acid, L-lactic acid, D-lactic acid, oxalic acid, L-mandelic acid and D-mandelic acid, and hydrates and solvates of said salts.
(3) Sitagliptin salt according to item (1), which is sitagliptin D-glucuronate, or a hydrate or solvate thereof, optionally being in crystalline form or in amorphous form.
(4) Sitagliptin D-glucuronate according to previous item being in crystalline form and having a powder X-ray diffraction pattern comprising the following characteristic reflection angles 20: 5.1±0.2°, 12.7±0.2°, 15.4±0.2°., 17.1±0.2°, 19.5±0.2°, 21.5±0.2°, 22.5±0.2°, 26.2±0.2° and 26.9±0.2°.
(5) Sitagliptin salt according to item (1), which is sitagliptin glutarate, or a hydrate or solvate thereof, optionally being in crystalline form or in amorphous form.
(6) Sitagliptin glutarate according to item (5) being in crystalline form and having a powder X-ray diffraction pattern comprising the following characteristic reflection angles 20: 6.4±0.2°, 8.0±0.2°, 12.9±0.2°, 15.4±0.2°, 17.6±0.2°, 20.8±0.2°, 23.0±0.2°, 24.7±0.2°, 25.4±0.2° and 26.6±0.2°.
(7) Sitagliptin salt according to item (1), which is sitagliptin hydrogen sulfate, or a hydrate or solvate thereof, optionally being in crystalline form or in amorphous form.
(8) Sitagliptin hydrogen sulfate according to item (7) being in crystalline form and having a powder X-ray diffraction pattern comprising the following characteristic reflection angles 2θ:4.7±0.2°, 14.2±0.2°, 15.4±0.2°, 18.1±0.2°, 19.3±0.2°, 22.0±0.2°, 23.3±0.2°, 24.4±0.2°, 26.3±0.2° and 26.8±0.2°.
(9) Sitagliptin salt according to item (1), which is sitagliptin L-lactate, or a hydrate or solvate thereof, optionally being in crystalline form or in amorphous form.
(10) Sitagliptin L-lactate according to item (9) being in crystalline form and having a powder X-ray diffraction pattern comprising the following characteristic reflection angles 2θ: 6.4±0.2°, 7.9±0.2°, 10.5±0.2°, 17.8±0.2°, 20.3±0.2°, 21.5±0.2°, 23.8±0.2°, 24.5±0.2°, 25.7±0.2° and 27.3±0.2°.
(11) Sitagliptin salt according to item (1), which is sitagliptin oxalate, or a hydrate or solvate thereof, optionally being in crystalline form or in amorphous form.
(12) Sitagliptin oxalate according to item (11) being in crystalline form and having a powder X-ray diffraction pattern comprising the following characteristic reflection angles 2θ: 8.4±0.2°, 11.2±0.2°, 17.0±0.2°, 17.5±0.2°, 18.4±0.2°, 20.9±0.2°, 23.9±0.2°, 25.4±0.2°, 27.0±0.2° and 27.9±0.2°.
(13) Process for the preparation of a sitalgiptin salt according to any one of items
   (1)-(12) comprising the following steps:
      a) providing a mixture comprising sitagliptin (base) and pharmaceutically acceptable acid selected from the group consisting of D-glucuronic acid, L-glucuronic acid, glutaric acid, sulfuric acid, L-lactic acid, D-lactic acid, oxalic acid, ethanesulfonic acid, acetic acid, L-mandelic acid, D-mandelic acid, capric acid, benzoic acid, hippuric acid, trans-cinnamic acid, malonic acid, citric acid, 1-hydroxy-2-naphtolic acid, crotonic acid and ascorbic acid.
      b) isolating the obtained sitagliptin salt.
(14) The process according to item (13), wherein said mixture is provided such that, after the sitagliptin base and selected pharmaceutically acceptable acid have been dissolved in a liquid medium, the solution is maintained, optionally with agitating, at a temperature lower than 40ºC., preferably lower than 30°C., until said sitagliptin salt is formed; whereupon the obtained sitagliptin salt is isolated from the liquid medium without increase of temperature to 40ºC or more.
(15) The process according to item (13) or (14), wherein after dissolution said mixture is kept until salt formation at a temperature of about 20 to 25ºC.
(16) A pharmaceutical composition comprising a sitagliptin salt according to any one of items (1)-(12).
(17) A pharmaceutical composition according to item (16), wherein said sitagliptin salt is sitagliptin D-glucuronate.
(18) A pharmaceutical composition according to item (16), wherein said sitagliptin salt is sitagliptin glutarate.
(19) A pharmaceutical composition according to item (16), wherein said sitagliptin salt is sitagliptin hydrogen sulfate.
(20) A pharmaceutical composition according to item (16), wherein said sitagliptin salt is sitagliptin L-lactate.
(21) A pharmaceutical composition according to item (16), wherein said sitagliptin salt is sitagliptin oxalate.
(22) A pharmaceutical composition according to any one of items (16) to (21), for use in therapeutic treatment of Type 2 diabetes in a mammal.

It must be emphasized that the existence and properties of individual salts are inherently unpredictable. Hence although numerous acids are available to try as alternatives, the skilled person cannot predict which, if any, is likely to provide a salt of sitagliptin having physical and/or chemical properties to make suitable to be included into a pharmaceutical composition, and if so, which one can provide appropriate dosage or administration forms. For example an active compound may in principle be used in tablets or capsules in neutral form or as magnesium salt, while for parenteral use sodium salt may be preferred. Sitagliptin is preferably used as hydrogenphosphate salt but it is well known that phosphates are easily precipitated by calcium and magnesium ions and therefore calcium or magnesium containing excipients may influence bioavailability. In addition simultaneous consuming of food rich on calcium may cause irregular bio-behaviour.
Thus there is a need to obtain new salts of sitagliptin that may have advantageous physico-chemical and biokinetic properties such as suitable solubility in neutral, acidic or alkaline water medium, solubility in technologically important organic solvents, water/lipid partition coefficient, electrochargeability, storage stability, thermal stability, water and oxygen inertness, hygroscopity, crystal shape, particle size and surface, dissolution profile, compatibility with excipients and combined active ingredients or special properties for final dosage form design. Special salts may have beneficial technological properties such as ease of purification and impurity removal. A better solubility in physiological conditions is surely one of most important properties, in particular for immediate release oral final dosage forms.

We have surprisingly found that using pharmaceutically acceptable ions selected from the group of D-glucuronic acid, L-glucuronic acid, glutaric acid, sulfuric acid, L-lactic acid, D-lactic acid, oxalic acid, ethanesulfonic acid, acetic acid, L-mandelic acid, D-mandelic acid, capric acid, benzoic acid, hippuric acid, trans-cinnamic acid, malonic acid, citric acid, 1-hydroxy-2-naphtolic acid, crotonic acid and ascorbic acid well defined salts of sitagliptin with specific physico-chemical properties are formed. Without being bound to any theory, it can be assumed that the particularly chosen ions form favorable associations with the beta amine group linked to the amido group of the sitagliptin structure, thereby controlling decomposition of the corresponding sitagliptin salt and hydrates and solvates thereof, yet enabling rapid dissolution in water or aqueous media.
With a view of specific physico-chemical properties in terms of both high water solubility and high stability, preferred salts of present invention are sitagliptin D-glucuronate, sitagliptin glutarate, sitagliptin hydrogen sulfate, sitagliptin L-lactate and sitagliptin oxalate, and hydrates and solvates of said salts. Respective salts in crystal form also provide solid states with characteristic X-ray diffraction patterns.
Especially when the pharmaceutically acceptable acid is selected from the group consisting of D-glucuronic acid, L-glucuronic acid, glutaric acid, sulfuric acid, L-lactic acid, D-lactic acid, oxalic acid, L-mandelic acid and D-mandelic acid (and hydrates and solvates of said salts) it is possible to obtain good overall characteristics of both good water solubility and high thermal stability, for example compared to sitagliptin dihydrogen phosphate. Moreover, sharing the characteristic of representing physiologically ubiquitous ions favors pharmaceutical acceptance of such salts.
Specifically, stability and in particular thermal stability of salts disclosed herein are relevant to control the tendency of generation of decomposition products, e.g. under conditions of processing, handling, storage, etc.
Particularly preferred salts according to present invention are stable and show very good solubility in water (sitagliptin D-glucuronate, sitagliptin glutarate, sitagliptin hydrogen sulfate, sitagliptin L-lactate and sitagliptin oxalate). In addition such salts are easy to handle and process and therefore suitable for the manufacture of various pharmaceutical dosage forms. Excellent solubility in water while still remaining thermally stable ensure valuable and useful performance for the resulting pharmaceutical composition.

### Detailed description of the Invention

In the following, the present invention will be described in more detail by preferred embodiments and examples while referring to the attached drawings, noting, however, that these embodiments, examples and drawings are presented for illustrative purposes only and shall not limit the invention in any way.

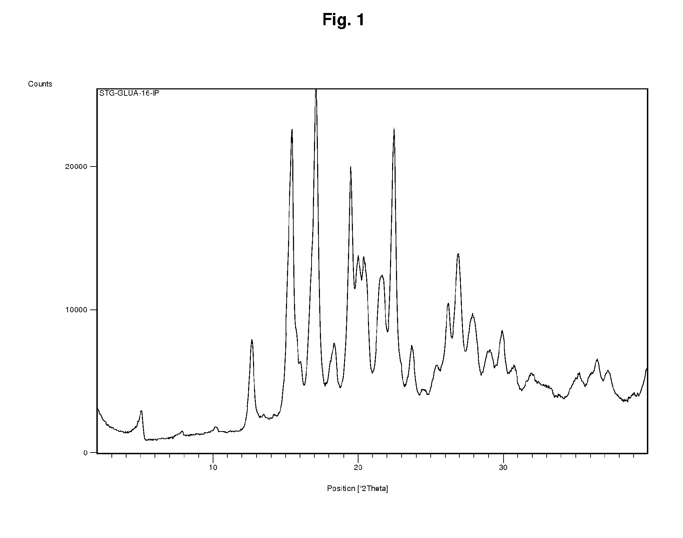

Figure 1 is a characteristic X-Ray diffraction pattern of crystalline sitagliptin D-glucuronate of present invention

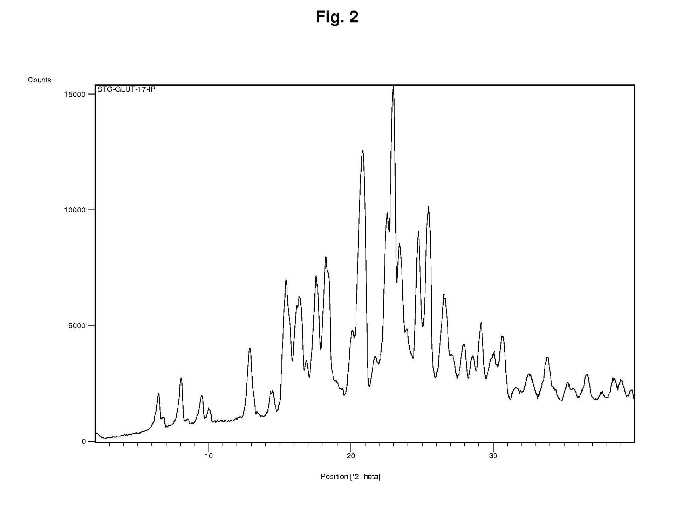

Figure 2 is a characteristic X-Ray diffraction pattern of crystalline sitagliptin glutarate of present invention

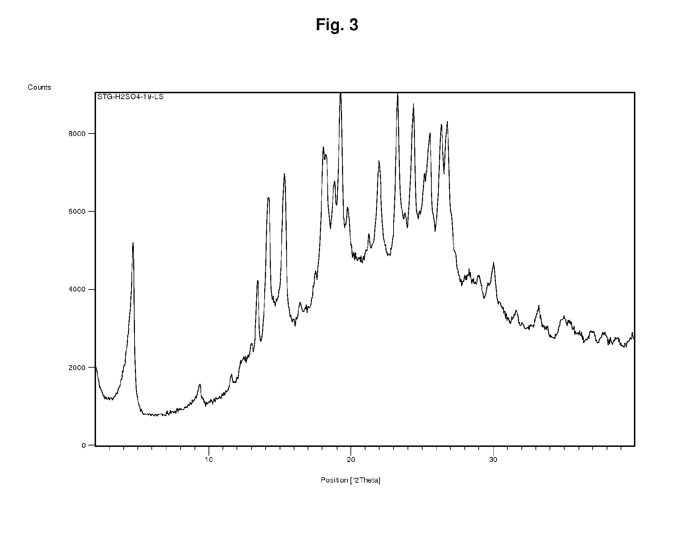

Figure 3 is a characteristic X-Ray diffraction pattern of crystalline sitagliptin hydrogen sulfate of present invention

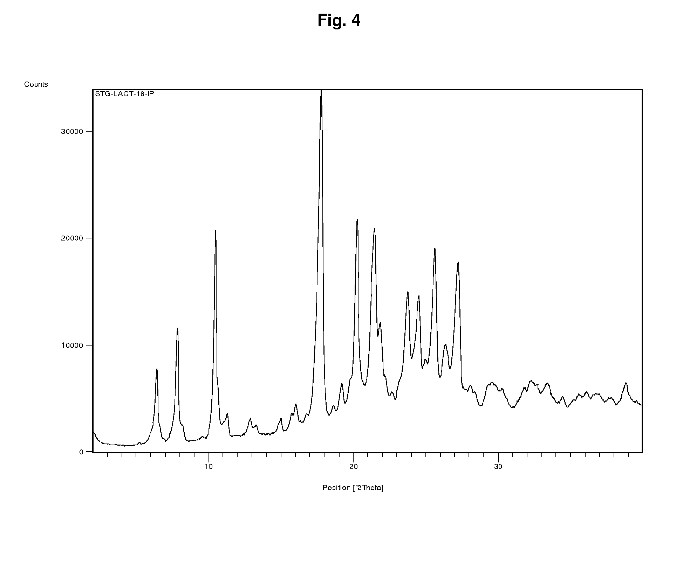

Figure 4 is a characteristic X-Ray diffraction pattern of crystalline sitagliptin L-lactate of present invention

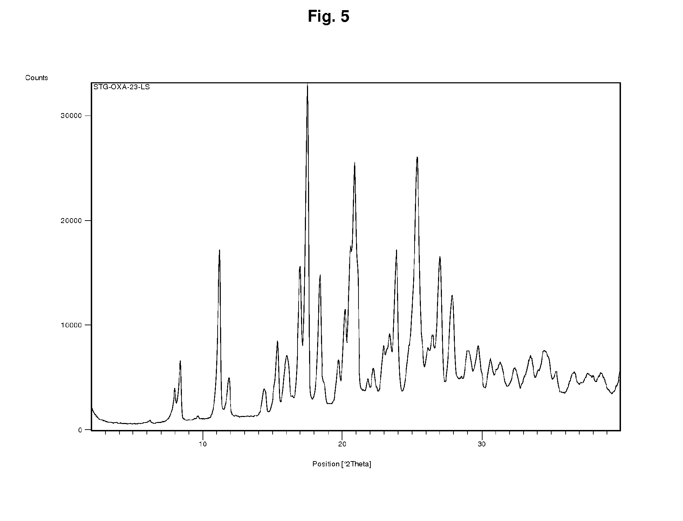

Figure 5 is a characteristic X-Ray diffraction pattern of crystalline sitagliptin oxalate of present invention

The present invention relates to novel acid salts of sitagliptin, wherein the acid is selected from the group consisting of D-glucuronic acid, L-glucuronic acid, glutaric acid, sulfuric acid, L-lactic acid, D-lactic acid, oxalic acid, ethanesulfonic acid, acetic acid, L-mandelic acid, D-mandelic acid, capric acid, benzoic acid, hippuric acid, trans-cinnamic acid, malonic acid, citric acid, 1-hydroxy-2-naphtolic acid, crotonic acid and ascorbic acid, and to hydrates and solvates of said acid salts.
Preferably pharmaceutically acceptable acid is selected from the group consisting of D-glucuronic acid, L-glucuronic acid, glutaric acid, sulfuric acid, L-lactic acid, D-lactic acid, oxalic acid, L-mandelic acid and D-mandelic acid, and hydrates and solvates of said salts. Particularly preferred salts according to present invention are sitagliptin D-glucuronate, sitagliptin glutarate, sitagliptin hydrogen sulfate, sitagliptin L-lactate and sitagliptin oxalate

In one aspect the present invention relates to sitagliptin D-glucuronate or hydrate or solvate thereof.

In another aspect the present invention relates to crystalline sitagliptin D-glucuronate. In another aspect the present invention relates to crystalline sitagliptin D-glucuronate having a powder X-ray diffraction pattern comprising the following characteristic reflection angles 2θ: 5.1±0.2°, 12.7±0.2°, 15.4±0.2°, 17.1±0.2°, 19.5±0.2°, 21.5±0.2° 22.5±0.2° 26.2±0.2° and 26.9±0.2°.
In yet another aspect the present invention relates to sitagliptin D-glucuronate in amorphous form.

In another aspect the present invention relates to sitagliptin glutarate or hydrate or solvate thereof.
In another aspect the present invention relates to crystalline sitagliptin glutarate.
In another aspect the present invention relates to crystalline sitagliptin glutarate having a powder X-ray diffraction pattern comprising the following characteristic reflection angles 2θ: 6.4±0.2° 8.0±0.2° 12.9±0.2° 15.4±0.2° 17.6±0.2° 20.8±0.2° 23.0±0.2° 24.7±0.2° 25.4±0.2° and 26.6±0.2°.
In yet another aspect the present invention relates to sitagliptin glutarate in amorphous form.

In another aspect the present invention relates to sitagliptin hydrogen sulfate or hydrate or solvate thereof.
In another aspect the present invention relates to crystalline sitagliptin hydrogen sulfate.
In another aspect the present invention relates to crystalline sitagliptin hydrogen sulfate having a powder X-ray diffraction pattern comprising the following characteristic reflection angles 2θ: 4.7±0.2°, 14.2±0.2°, 15.4±0.2°, 18.1±0.2°, 19.3±0.2°, 22.0±0.2°, 23.3±0.2°, 24.4±0.2°, 26.3±0.2° and 26.8±0.2°.
In yet another aspect the present invention relates to sitagliptin hydrogen sulfate in amorphous form.

In another aspect the present invention relates to sitagliptin L-lactate or hydrate or solvate thereof.
In another aspect the present invention relates to crystalline sitagliptin L-lactate.

In another aspect the present invention relates to crystalline sitagliptin L-lactate having a powder X-ray diffraction pattern comprising the following characteristic reflection angles 2θ: 6.4±0.2°, 7.9±0.2°, 10.5±0.2°, 17.8(0.2°, 20.3(0.2°, 21.5(0.2°, 23.8±0.2°, 24.5±0.2°, 25.7±0.2° and 27.3±0.2°.
In yet another aspect the present invention relates to sitagliptin L-lactate in amorphous form.

In another aspect the present invention relates to sitagliptin oxalate or hydrate or solvate thereof.
In another aspect the present invention relates to crystalline sitagliptin oxalate.
In another aspect the present invention relates to crystalline sitagliptin oxalate having a powder X-ray diffraction pattern comprising the following characteristic reflection angles 2θ: 8.4±0.2°, 11.2±0.2°, 17.0±0.2°, 17.5±0.2°, 18.4±0.2°, 20.9±0.2°, 23.9±0.2°, 25.4±0.2°, 27.0±0.2° and 27.9±0.2°.
In yet another aspect the present invention relates to sitagliptin oxalate in amorphous form.

In another aspect the present invention relates to a process of preparing salts of sitagliptin with pharmaceutically acceptable acids selected from the group of D-glucuronic acid, L-glucuronic acid, glutaric acid, sulfuric acid, L-lactic acid, D-lactic acid, oxalic acid, ethanesulfonic acid, acetic acid, L-mandelic acid, D-mandelic acid, capric acid, benzoic acid, hippuric acid, trans-cinnamic acid, malonic acid, citric acid, 1-hydroxy-2-naphtolic acid, crotonic acid and ascorbic acid by providing a mixture of sitagliptin base and pharmaceutically acceptable acid selected from the group of D-glucuronic acid, L-glucuronic acid, glutaric acid, sulfuric acid, L-lactic acid, D-lactic acid, oxalic acid, ethanesulfonic acid, acetic acid, L-mandelic acid, D-mandelic acid, capric acid, benzoic acid, hippuric acid, trans-cinnamic acid, malonic acid, citric acid, 1-hydroxy-2-naphtolic acid, crotonic acid and ascorbic acid respectively in a suitable solvent system, comprised of a single solvent or a mixture of solvents, and isolating the obtained sitagliptin salt by precipitation, filtration of the solid salt, evaporation, spray drying or other conventional techniques known in the art.

Suitable solvents are solvents selected from alcohols, ketones, nitriles, esters and water or mixtures thereof, preferably selected from acetone, C₁-C₄ alcohols (preferably selected from methanol, ethanol and isopropyl alcohol (iPrOH)), acetonitrile and water or mixtures thereof.

Pharmaceutically acceptable acid in natural state or in solution can be added to the solution of sitagliptin base.

Pharmaceutically acceptable acid is preferably added in an equimolar ratio to sitagliptin base, or an excess of the acid is used.

The temperature of solvent system comprising a mixture of sitagliptin base and pharmaceutically acceptable acid is from ambient temperature to the boiling point of the solvent system. After the sitagliptin base and the selected organic acid have been dissolved in a liquid medium, the obtained solution is preferably kept at a temperature lower than 40 ºC., more preferably lower than 30ºC., suitably at around ambient temperature such as about 20 to 25ºC., until the salt is formed. Paying attention to such low temperature conditions contributes to obtain and maintain a stable salt form without otherwise risking the generation of decomposition products. It has been found that considerable amounts of decomposition or disintegration products appear with the salts of the present invention in solution at higher temperatures.

Sitagliptin salt can be isolated or recovered from the reaction solution by precipitation. The precipitation can be spontaneous depending on solvent system. Alternatively, the precipitation can be induced by reducing the temperature of reaction mixture, especially if initial temperature of reaction mixture is elevated. The precipitation can also be induced by reduction of solution volume, preferably under diminished pressure, or by complete evaporation of solvent. Furthermore, the precipitation may be caused by adding an antisolvent, e.g. water, ethers and hydrocarbons. Alternatively precipitation may be initiated by adding crystallization seeds.

In one aspect of the invention sitagliptin salt is prepared by adding pharmaceutically acceptable acid in natural state or in solution to the solution of sitagliptin in a solvent selected from alcohols, ketones, nitriles, esters and water or mixtures thereof, preferably selected from acetone, C₁-C₄ alcohols, acetonitrile and water, optionally heating the mixture to obtain a solution and cooling. The precipitation of salt occurs after long standing the solution at appropriate temperature below 40 ºC., preferably between -10 to 25 ºC after cooling the stirred mixture from heated solution below 40 ºC., preferably to room temperature, such as about 20 to 25°C, or below (while optionally adding crystallization seeds), both after optional concentration of the solution by partial evaporation of solvents.
In another option the salt is formed by reprecipitation in a suspension of one or both starting components, or by precipitation adding antisolvent preferably selected from water, ethers and hydrocarbons, most preferably from water and diethyl ether. Isopropyl alcohol (iPrOH) may also be used as antisolvent.

In another aspect of the invention sitagliptin salts are prepared by adding pharmaceutically acceptable acid in natural state or dissolved to a solution of sitagliptin base in lower alcohol preferably methanol, ethanol or isopropyl alcohol (iPrOH), following by complete or partial evaporation of the solvents.

In another aspect of the present invention sitagliptin D-glucuronate, sitagliptin L-glucuronate, sitagliptin glutarate, sitagliptin hydrogen sulfate, sitagliptin L-lactate, sitagliptin D-lactate, sitagliptin oxalate, sitagliptin ethanesulfonate, sitagliptin acetate, sitagliptin L-mandelate, sitagliptin D-mandelate, sitagliptin caprate, sitagliptin benzoate, sitagliptin hippurate, sitagliptin trans-cinnamate, sitagliptin malonate, sitagliptin citrate, sitagliptin 1-hydroxy-2-naphtolate, sitagliptin crotonate or sitagliptin ascorbate is prepared by adding D-glucuronic acid, L-glucuronic acid, glutaric acid, sulfuric acid, L-lactic acid, D-lactic acid, oxalic acid, ethanesulfonic acid, acetic acid, L-mandelic acid, D-mandelic acid, capric acid, benzoic acid, hippuric acid, trans-cinnamic acid, malonic acid, citric acid, 1-hydroxy-2-naphtolic acid, crotonic acid or ascorbic acid in solid state or in solution to the solution of sitagliptin in a solvent selected from alcohols, ketones, nitriles, esters and water or mixtures thereof, preferably selected from acetone, C₁-C₄ alcohols, acetonitrile and water, optionally heating the mixture to obtain a solution and cooling. The precipitation of salt occurs after long standing the solution at appropriate temperature below 40 ºC., preferably between -10 to 25 ºC after cooling the stirred mixture from heated solution below 40 ºC., preferably to room temperature, such as about 20 to 25°C, or below (while optionally adding crystallization seeds), both after optional concentration of the solution by partial evaporation of solvents. In another option the salt is formed by reprecipitation in a suspension of one or both starting components, or by precipitation adding antisolvent preferably selected from water, ethers and hydrocarbons. Isopropyl alcohol (iPrOH) may also be used as antisolvent.

In one preferred example sitagliptin base is dissolved methanol. D-glucuronic acid in a solid state is added to the solution of sitagliptin base under stirring and typically at around room temperature, such as about 20 to 25°C, preferably in an equimolar ratio to sitagliptin base or in a slight excess. When homogenous solution is formed, isopropyl alcohol (iPrOH) is slowly added under stirring typically at around room temperature. The dispersion is subsequently left to stir for 6 - 24 hours, preferably for about 12 hours at about room temperature. The obtained crystals are collected, preferably using suction filtration through a porous ceramic filter, and optionally washed with isopropyl alcohol (iPrOH).
Sitagliptin D-glucuronate prepared according to such procedure exhibits powder X-ray diffraction pattern comprising the following characteristic reflection angles 2θ: 5.1±0.2°, 12.7±0.2°, 15.4±0.2°, 17.1±0.2°, 19.5±0.2°, 21.5±0.2°, 22.5±0.2°, 26.2±0.2° and 26.9±0.2°.
Sitagliptin D- glucuronate prepared according to such procedure exhibits melting point at about 126 to 129 °C.

In another preferred example sitagliptin base is dissolved in acetonitrile. Solution of glutaric acid, preferably in acetonitrile, is added dropwise to the solution of sitagliptin base under stirring and typically at around room temperature, such as about 20 to 25ºC, preferably in an equimolar ratio to sitagliptin base or in a slight excess. The solution mixture is afterwards heated to reflux temperature until clear solution is formed and subsequently left to slowly cool, preferably to around room temperature under stirring. Optionally crystallization seeds are added while cooling the solution.

Obtained crystals are collected, preferably using suction filtration through a porous ceramic filter.
Said crystallization seeds are prepared by dissolving sitagliptin free base in acetonitrile. Solution of glutaric acid, preferably in acetonitrile, is added dropwise to the solution of sitagliptin base under stirring and typically at around room temperature, preferably in an equimolar ratio to sitagliptin base, and subsequently solvent is evaporated. Sitagliptin glutarate prepared according to such procedure exhibits a powder X-ray diffraction pattern comprising the following characteristic reflection angles 2θ: 6.4±0.2°, 8.0±0.2°, 12.9±0.2°, 15.4±0.2°, 17.6±0.2°, 20.8±0.2°, 23.0±0.2°, 24.7±0.2°, 25.4±0.2 and 26.6±0.2°.
Sitagliptin glutarate prepared according to such procedure exhibits melting point at about 112 to 118 °C.

In another preferred example sitagliptin base is dissolved in acetonitrile. Solution of sulfuric acid (preferably concentrated 95 - 97% sulfuric acid is used), preferably in acetonitrile, is added dropwise to the solution of sitagliptin base under stirring and typically at around room temperature, such as about 20 to 25θºC, preferably in an equimolar ratio to sitagliptin base or in a slight excess. The solution mixture is afterwards left to stir, preferably for about 2 hours typically at around room temperature. Subsequently solvent is evaporated and formed solid is dispersed, preferably in methyl tert-butyl ether (tBuOMe), optionally under stirring typically at around room temperature, preferably for about 2 hours. Obtained crystals are collected, preferably using suction filtration through a porous ceramic filter. Sitagliptin hydrogen sulfate prepared according to such procedure exhibits a powder X-ray diffraction pattern comprising the following characteristic reflection angles 2θ: 4.7±0.2°, 14.2±0.2°, 15.4±0.2°, 18.1±0.2°, 19.3±0.2°, 22.0±0.2°, 23.3±0.2°, 24.4±0.2°, 26.3±0.2° and 26.8±0.2°.
Sitagliptin hydrogen sulfate prepared according to such procedure exhibits melting point at about 184 to 190 °C.

In another preferred example sitagliptin base is dissolved in mixture of methanol and isopropyl alcohol (iPrOH), preferably in volumetric ratio of about 1 : 5, respectively. L-lactic acid, preferably concentrated 99 % L-lactic acid is used, is added dropwise to the solution of sitagliptin base typically at around room temperature, such as about 20 to 25ºC, and optionally under stirring, preferably in an equimolar ratio to sitagliptin base or in a slight excess. Afterwards crystallization seeds are added. Obtained dispersion is subsequently left to stir for 6 - 24 hours, preferably for about 12 hours at about room temperature. The obtained crystals are collected, preferably using suction filtration through a porous ceramic filter.
Said crystallization seeds are prepared by dissolving sitagliptin free base in mixture of methanol and isopropyl alcohol (iPrOH), preferably in volumetric ratio of about 1 : 5, respectively. L-lactic acid, preferably concentrated 99 % L-lactic acid is used, is added dropwise to the solution of sitagliptin base typically at around room temperature and optionally under stirring, preferably in an equimolar ratio to sitagliptin base, and subsequently solvent is evaporated.
Sitagliptin L-lactate prepared according to such procedure exhibits a powder X-ray diffraction pattern comprising the following characteristic reflection angles 2θ: 6.4±0.2°, 7.9±0.2°, 10.5±0.2°, 17.8±0.2°, 20.3±0.2°, 21.5±0.2°, 23.8±0.2°, 24.5±0.2°, 25.7±0.2 and 27.3±0.2°.
Sitagliptin L-lactate prepared according to such procedure exhibits melting point at about 150 to 151 °C.

In another preferred example sitagliptin base is dissolved in ethanol. Oxalic acid in a solid state is added to the solution of sitagliptin base under stirring and typically at around room temperature, such as about 20 to 25°C, preferably in an equimolar ratio to sitagliptin base or in a slight excess. The solution is left to stir until precipitate is formed. The obtained crystals are collected, preferably using suction filtration through a porous ceramic filter. Sitagliptin oxalate prepared according to such procedure exhibits a powder X-ray diffraction pattern comprising the following characteristic reflection angles 2θ: 8.4±0.2°, 11.2±0.2°, 17.0±0.2°, 17.5±0.2°, 18.4±0.2°, 20.9±0.2°, 23.9±0.2°, 25.4±0.2°, 27.0±0.2 and 27.9±0.2°.
Sitagliptin oxalate prepared according to such procedure exhibits melting point at about 189 to 190 °C.

In another preferred example sitagliptin base is dissolved in mixture of methanol and isopropyl alcohol (iPrOH), preferably in volumetric ratio of about 1 : 6, respectively. Water solution of ethanesulfonic acid, preferably 0.2 M solution of ethanesulfonic acid, is added dropwise to the solution of sitagliptin base under stirring and typically at around room temperature, such as about 20 to 25ºC, preferably in an equimolar ratio to sitagliptin base or in a slight excess. The solution mixture is afterwards left to stir, preferably for about 2 hours typically at around room temperature. The obtained crystals of sitagliptin ethanesulfonate are collected, preferably using suction filtration through a porous ceramic filter.
Sitagliptin ethanesulfonate prepared according to such procedure exhibits melting point at about 199 to 202 °C.

In another preferred example sitagliptin base is dissolved in methanol. Acetic acid, preferably concentrated 100 % acetic acid, is added dropwise to the solution of sitagliptin base under stirring and typically at around room temperature, such as about 20 to 25ºC, preferably in an equimolar ratio to sitagliptin base or in a slight excess. Afterwards solvent is evaporated and formed solid is dispersed, preferably in isopropyl alcohol (iPrOH), and subsequently heated to around 70°C until the entire residue is dissolved. The solution is then put in freezer, preferably at the temperature below -5°C, for 6 - 24 hours, preferably for about 12 hours. The obtained crystals of sitagliptin acetate are collected, preferably using suction filtration through a porous ceramic filter.
Sitagliptin acetate prepared according to such procedure exhibits melting point at about 113 to 117 °C.

In another preferred example sitagliptin base is dissolved in acetonitrile. Capric acid in a solid state is added to the solution of sitagliptin base typically at around room temperature, such as about 20 to 25ºC, and optionally under stirring, preferably in an equimolar ratio to sitagliptin base or in a slight excess. The solution is left to stir for about 1 hour and subsequently solvent is evaporated. The solid sitagliptin caprate is optionally further dried for 6 - 24 hours, preferably for about 12 hours, at reduced pressure at around 30ºC.

Sitagliptin caprate prepared according to such procedure exhibits melting point at about 93 to 97 °C.

In yet another preferred example sitagliptin base is dissolved in ethanol. L-mandelic acid in a solid state is added to the solution of sitagliptin base optionally under stirring and typically at around room temperature, such as about 20 to 25ºC, preferably in an equimolar ratio to sitagliptin base or in a slight excess. Subsequently solvent is evaporated and sitagliptin L-mandelate as white powder is obtained.
Sitagliptin L-mandelate prepared according to such procedure exhibits melting point at about 166 to 170 °C.

Another aspect of the present invention is a pharmaceutical composition for administering a therapeutically effective amount of sitagliptin salts with pharmaceutically acceptable acids of the present invention, i.e. sitagliptin D-glucuronate, sitagliptin L-glucuronate, sitagliptin glutarate, sitagliptin hydrogen sulfate, sitagliptin L-lactate, sitagliptin D-lactate, sitagliptin oxalate, sitagliptin ethanesulfonate, sitagliptin acetate, sitagliptin L-mandelate, sitagliptin D-mandelate, sitagliptin caprate, sitagliptin benzoate, sitagliptin hippurate, sitagliptin trans-cinnamate, sitagliptin malonate, sitagliptin citrate, sitagliptin 1-hydroxy-2-naphtolate, sitagliptin crotonate or sitagliptin ascorbate, preferably sitagliptin D-glucuronate, sitagliptin glutarate, sitagliptin hydrogen sulfate, sitagliptin L-lactate or sitagliptin oxalate, in unit dosage form with one or more pharmaceutically acceptable carriers or other excipients.

A therapeutically effective amount of sitagliptin salt of the present invention is amount of salt ranging, when calculated as sitagliptin base, from 5 to 200 mg, preferably from 10 to 150 mg, more preferably from 25 to 100 mg.

Pharmaceutical acceptable salts in accordance with present invention can be embodied for example in form of tablet, capsules, pellets, granules and suppositories or their combined forms. Pharmaceutical composition in accordance with present invention can be suitable for immediate release or modified release of sitagliptin salts of the present invention. Solid pharmaceutical compositions can be for example coated with aim of increasing peletibility or regulating the disintegration or absorption.

Pharmaceutically acceptable excipients may be selected from the group consisting of binders, diluents, disintegrating agents, stabilizing agents, preservatives, lubricants, fragrances, flavoring agents, sweeteners and other excipients known in the field of the pharmaceutical technology. Preferably, carriers and excipients may be selected from the group consisting of lactose, microcrystalline cellulose, cellulose derivatives, (e.g. hydroxypropylcellulose, croscarmellose sodium), polyacrylates, calcium carbonate, starch, colloidal silicone dioxide, anhydrous dibasic calcium phosphate, sodium starch glycolate, talc, magnesium stearate, sodium stearyl fumarate, mannitol, polyvinylpyrrolidone, polyethylene glycol and other excipients known in the field of the pharmaceutical technology.

Optionally, the pharmaceutical compositions of the invention may be combination products comprising one or more additional pharmaceutically active components in addition to sitagliptin salts, preferably one or more additional pharmaceutically active components are selected from the group consisting of insulin sensitizers, insulin, insulin mimetics, sulfonylureas, α-glucosidase inhibitors, glucagon receptor antagonists, GLP-1, GLP-1 analogues, GLP-1 mimetics, GLP-1 receptor agonists, GIP, GIP mimetics, PACAP, PACAP mimetics, PACAP receptor agonists, cholesterol lowering agents, PPARδ agonists, antiobesity compounds, ileal bile acid tranporter inhibitors, agents intended for use in inflammatory conditions, antihypertensive agents, glucokinase activators (GKAs), inhibitors of 11 β-hydroxysteroid dehydrogenase type 1, inhibitors of cholesteryl ester transfer protein (CETP) and inhibitors of fructose 1,6-bisphosphatase.
Most preferably additional pharmaceutically active component is metformin and / or its pharmaceutically acceptable salt.

The pharmaceutical compositions according to the present invention may be prepared by methods known in the field of the pharmaceutical technology. In view of the temperature sensitivity of the sitagliptin salts disclosed herein, a preferred embodiment of the process for the preparation of a pharmaceutical composition according to the present invention however is one where all steps involving processing of the sitagliptin salt to obtain the desired final pharmaceutical composition are performed at a temperature lower than 40ºC, preferably lower than 30ºC. This particularly applies to steps performed in solution or under wet conditions. As a result, the respective sitagliptin salt can maintain stability and produce less decomposition products. Stability and production of less decomposition products can be defined by respective melting points disclosed herein.

The further aspect of the present invention is a method for treatment and/or prevention in mammal of clinical conditions for which DPP-IV inhibitor is indicated, in particular treatment of Type 2 diabetes, hyperglycemia, insulin reistance, and obesity, with a medicament by using an effective amount of sitagliptin salts according to the present invention, i.e. sitagliptin D-glucuronate, sitagliptin L-glucuronate, sitagliptin glutarate, sitagliptin hydrogen sulfate, sitagliptin L-lactate, sitagliptin D-lactate, sitagliptin oxalate, sitagliptin ethanesulfonate, sitagliptin acetate, sitagliptin L-mandelate, sitagliptin D-mandelate, sitagliptin caprate, sitagliptin benzoate, sitagliptin hippurate, sitagliptin trans-cinnamate, sitagliptin malonate, sitagliptin citrate, sitagliptin 1-hydroxy-2-naphtolate, sitagliptin crotonate or sitagliptin ascorbate, preferably sitagliptin D-glucuronate, sitagliptin glutarate, sitagliptin hydrogen sulfate, sitagliptin L-lactate or sitagliptin oxalate

In another aspect the present invention is related to use of sitagliptin salts according to present invention, i.e. sitagliptin D-glucuronate, sitagliptin L-glucuronate, sitagliptin glutarate, sitagliptin hydrogen sulfate, sitagliptin L-lactate, sitagliptin D-lactate, sitagliptin oxalate, sitagliptin ethanesulfonate, sitagliptin acetate, sitagliptin L-mandelate, sitagliptin D-mandelate, sitagliptin caprate, sitagliptin benzoate, sitagliptin hippurate, sitagliptin trans-cinnamate, sitagliptin malonate, sitagliptin citrate, sitagliptin 1-hydroxy-2-naphtolate, sitagliptin crotonate or sitagliptin ascorbate, preferably sitagliptin D-glucuronate, sitagliptin glutarate, sitagliptin hydrogen sulfate, sitagliptin L-lactate or sitagliptin oxalate for the manufacture of medicament for treatment and/or prevention in mammal of clinical conditions for which DPP-IV inhibitor is indicated, in particular treatment of Type 2 diabetes, hyperglycemia, insulin resistance, and obesity.

Experimental Procedures

**Table 1: Solubility of sitagliptin salts in water at 25°C.**

| 25°C | Dihydrogen phosphate | D-Glucuronate | Glutarate | Hydrogen sulfate | L-lactate |
|---|---|---|---|---|---|
| mg/ml (water) | 100 -120 | >500 | >500 | >300 | >300 |

The solubility in water was measured using Crystal16™ from Avantium Technologies.
Sitagliptin phosphate was prepared according to the procedure described in WO 2005/003135.

Sitagliptin free base was prepared according to the procedures of WO 2003/004498.

### Example 1 (Sitagliptin D-glucuronate):

In a 100 ml flask sitagliptin free base (1.20 g) and D-glucuronic acid (604 mg) were dissolved in methanol (8 ml) and water (3 ml) under stirring. When homogenous solution formed, iPrOH (isopropyl alcohol) (50 ml) was slowly added under stirring at room temperature. White precipitate formed and dispersion was left to stir for 12 hours at room temperature. Formed crystals where collected using suction filtration through a porous ceramic filter and washed with 10 ml of iPrOH to yield a white powder (1.64 g, 92%). Melting point 126 -129°C.

### Example 2 (Sitagliptin glutarate):

In a 50 ml flask sitagliptin free base (1.01 g) was dissolved in acetonitrile (10 ml). Then solution of glutaric acid (344 mg) in acetonitrile (7 ml) was added dropwise under stirring. After addition, precipitation appeared together with a gummy residue. Mixture was then heated to reflux temperature until clear solution formed and left to cool slowly to room temperature under stirring with addition of crystallization seeds.
Formed crystals where collected using suction filtration through a porous ceramic filter to yield a white powder (1.20 g, 90%). Melting point 112 -118°C.
Crystallization seeds used in Example 2 were prepared by dissolving sitagliptin free base (1.01 g) in acetonitrile (10 ml), adding dropwise glutaric acid (344 mg) in acetonitrile (7 ml) under stirring and subsequently evaporating the solvent.

### Example 3 (Sitagliptin hydrogensulfate):

In a 50 ml flask sitagliptin free base (505 mg) was dissolved in acetonitrile (10 ml). To this solution a solution of H₂SO₄ (66.4 µl, concentration = 95-97%) in acetonitrile (5 ml) was added drop wise and left to stir over 2 hours. After unsuccessful trial of filtration solution was evaporated to dryness and formed solid dispersed in methyl tert-butyl ether (tBuOMe) (50 ml) under stirring at room temperature for 2 hours.
Formed dispersion was filtered through a porous ceramic filter and white crystals where collected (592 mg, 95%). Melting point 184 -190°C.

### Example 4 (Sitagliptin L-lactate):

In a 50 ml flask sitagliptin free base (1.20 g) was dissolved in methanol (2 ml) and iPrOH (10 ml). Then *L*-lactic acid (243 µl, concentration = 99 %) was added dropwise. Crystallization seeds where added and precipitation started. The dispersion was left to stir at room temperature for 12 hours. Formed crystals where collected using suction filtration through a porous ceramic filter to yield a white powder (571 mg, 35%). Melting point 150 -151 °C.
Crystallization seeds used in Example 4 were prepared by dissolving sitagliptin free base (1.20 g) in methanol (2 ml) and iPrOH (10 ml), adding *L*-lactic acid (243 µl, concentration = 99 %) dropwise under stirring and subsequently evaporating the solvent.

### Example 5 (Sitagliptin oxalate):

To a solution of sitagliptin free base (1.05 g), dissolved in ethanol (15 ml), oxalic acid (0.23 g) was added and the solution was stirred at room temperature until precipitate was formed. Crystals (1.05g, 85%) were collected by filtration. Melting point 189 - 190 °C.

### Example 6 (Sitagliptin ethanesulfonate):

In a 50 ml flask sitagliptin free base (303 mg) was dissolved in methanol (1.5 ml) and iPrOH (10 ml). Then 0.2M solution of ethanesulfonic acid (3.72 ml) was added dropwise and left to stir for 2 hours at room temperature. Formed crystals were collected using suction filtration through a porous ceramic filter to yield a white powder (272 mg, 71 %). Melting point 199 -202°C.

### Example 7 (Sitagliptin acetate):

In a 50 ml flask sitagliptin free base (500 mg) was dissolved in 3ml methanol and acetic acid (81.7 µl, concentration = 100%) was added drop wise under stirring at room temperature. Solvent was evaporated and 9 ml of iPrOH was added to the flask. The flask was heated to ca. 70°C until the entire residue dissolved and was subsequently put in freezer for 12 hours. Formed crystals where collected using suction filtration through a porous ceramic filter as white powder (505 mg, 88%). Melting point 113 -117°C.

### Example 8 (Sitagliptin caprate):

In a 50 ml flask sitagliptin free base (307 mg) was dissolved in acetonitrile (10 ml) and capric acid (130 mg) was added. After the reaction mixture was left to stir for 1 hour at room temperature, solvent was evaporated. The solid residue was further dried over night (at reduced pressure of 5 mBar, 30°C). White powder was collected (437mg). Melting point 93 -97°C.

### Example 9 (Sitagliptin L-mandelate):

Sitagliptin free base (0.54g) and L-mandelic acid (0.20 g) were dissolved in ethanol (20 m). Solution was evaporated to give white powder (0.70g, 95%). Melting point 166 -170°C.
Other sitagliptin salts, such as sitagliptin L-malate, sitagliptin citrate, sitagliptin malonate, sitagliptin D-mandelate, sitagliptin benzoate and sitagliptin 1-hydroxy-2-naphtoate were prepared according to procedures similar to examples 1-9.

### Methods of analysis

X-Ray powder diffraction method:
Conditions for obtaining powder X-ray diffraction (XRD) patterns: The powder X-ray diffraction patterns were obtained by methods known in the art using Philips X'Pert PRO diffractometer with X'Celerator detector using CuKα radiation (tube operating at 45 kV and 40 mA) in the Bragg-Brentano (reflection) geometry. Data were recorded from 2 to 40 º2θ in steps of 0.033 º2θ and the measurement time of 50 seconds per step. Variable divergence and antiscatter slits were used to maintain 12 mm of sample length irradiated.

## Claims

1. Sitagliptin salt with a pharmaceutically acceptable acid, the pharmaceutically acceptable acid being selected from the group consisting of D-glucuronic acid, L-glucuronic acid, glutaric acid, sulfuric acid, L-lactic acid, D-lactic acid, ethanesulfonic acid, oxalic acid, acetic acid, L-mandelic acid, D-mandelic acid, capric acid, benzoic acid, hippuric acid, trans-cinnamic acid, malonic acid, citric acid, 1-hydroxy-2-naphtolic acid, crotonic acid and ascorbic acid, and hydrates and solvates of said salts.

2. Sitagliptin salt according to claim 1, wherein the pharmaceutically acceptable acid is selected from the group consisting of D-glucuronic acid, L-glucuronic acid, glutaric acid, sulfuric acid, L-lactic acid, D-lactic acid, oxalic acid, L-mandelic acid and D-mandelic acid, and hydrates and solvates of said salts.

3. Sitagliptin salt according to claim 1, which is sitagliptin D-glucuronate, or a hydrate or solvate thereof, optionally being in crystalline form or in amorphous form.

4. Sitagliptin D-glucuronate according to claim 3 being in crystalline form and having a powder X-ray diffraction pattern comprising the following characteristic reflection angles 2θ: 5.1±0.2°, 12.7±0.2°, 15.4±0.2°, 17.1±0.2°, 19.5±0.20, 21.5±0.2°, 22.5±0.2°, 26.2±0.2 ° and 26.9±0.2°.

5. Sitagliptin salt according to claim 1, which is sitagliptin glutarate, or a hydrate or solvate thereof, optionally being in crystalline form or in amorphous form.

6. Sitagliptin glutarate according to claim 5 being in crystalline form and having a powder X-ray diffraction pattern comprising the following characteristic reflection angles 2θ: 6.4±0.2°, 8.0±0.2°, 12.9±0.2°, 15.4±0.2°, 17.6±0.2°, 20.8±0.2°, 23.0±0.2°, 24.7±0.2°, 25.4±0.2° and 26.6±0.2°.

7. Sitagliptin salt according to claim 1, which is sitagliptin hydrogen sulfate, or a hydrate or solvate thereof, optionally being in crystalline form or in amorphous form.

8. Sitagliptin hydrogen sulfate according to claim 7 being in crystalline form and having a powder X-ray diffraction pattern comprising the following characteristic reflection angles 2θ: 4.7±0.2°, 14.2±0.2°, 15.4±0.2°, 18.1±0.2°, 19.3±0.2°, 22.0±0.2°, 23.3±0.2°, 24.4±0.2°, 26.3±0.2 ° and 26.8±0.2°.

9. Sitagliptin salt according to claim 1, which is sitagliptin L-lactate, or a hydrate or solvate thereof, optionally being in crystalline form or in amorphous form.

10. Crystalline Sitagliptin L-lactate according to claim 9 being in crystalline form and having a powder X-ray diffraction pattern comprising the following characteristic reflection angles 2θ: 6.4±0.2°, 7.9±0.2°, 10.5±0.2°, 17.8±0.2°, 20.3±0.2°, 21.5±0.2°, 23.8±0.2°, 24.5±0.2°, 25.7±0.2 ° and 27.3±0.2°.

11. Sitagliptin salt according to claim 1, which is sitagliptin oxalate, or a hydrate or solvate thereof, optionally being in crystalline form or in amorphous form.

12. Sitagliptin oxalate according to claim 11 being in crystalline form and having a powder X-ray diffraction pattern comprising the following characteristic reflection angles 2θ: 8.4±0.2°, 11.2±0.2°, 17.0±0.2°, 17.5±0.2°, 18.4±0.2°, 20.9±0.2°, 23.9±0.2°, 25.4±0.2°, 27.0±0.2 ° and 27.9±0.2°.

13. Process for the preparation of a sitalgiptin salt according to any one of claims 1-12 comprising the following steps:
a) providing a mixture comprising sitagliptin (base) and pharmaceutically acceptable acid selected from the group consisting of D-glucuronic acid, L-glucuronic acid, glutaric acid, sulfuric acid, L-lactic acid, D-lactic acid, ethanesulfonic acid, oxalic acid, acetic acid, L-mandelic acid, D-mandelic acid, capric acid, benzoic acid, hippuric acid, trans-cinnamic acid, malonic acid, citric acid, 1-hydroxy-2-naphtolic acid, crotonic acid and ascorbic acid, preferably selected from the group consisting of D-glucuronic acid, L-glucuronic acid, glutaric acid, sulfuric acid, L-lactic acid, D-lactic acid, oxalic acid, L-mandelic acid and D-mandelic acid; and
b) isolating the obtained sitagliptin salt.

14. The process according to claim 13, wherein said mixture is provided such that, after the sitagliptin base and selected pharmaceutically acceptable acid have been dissolved in a liquid medium, the solution is maintained, optionally with agitating, at a temperature lower than 40°C, preferably lower than 30°C, until said sitagliptin salt is formed; whereupon the obtained sitagliptin salt is isolated from the liquid medium without increase of temperature to 40°C or more.

15. The process according to claim 13 or 14, wherein after dissolution said mixture is kept until salt formation at a temperature of about 20 to 25 °C.

16. A pharmaceutical composition comprising a sitagliptin salt according to any one of claims 1 to 12.

17. A pharmaceutical composition according to claim 16, wherein said sitagliptin salt is sitagliptin D-glucuronate, sitagliptin glutarate, sitagliptin hydrogen sulfate, sitagliptin L-lactate, or sitagliptin oxalate.

18. A pharmaceutical composition according to claim 16 or 17, for use in therapeutic treatment of Type 2 diabetes in mammal.
